# EUROPÄISCHE PATENTANMELDUNG

(11) **EP 4 806 469 A2**
(43) Veröffentlichungstag der Anmeldung: **16.09.2026**
(21) Anmeldenummer: 26163867.0
(22) Anmeldetag: 11.03.2026
(51) Int. Cl.: A61L 2/208, B65B 55/10, A61L 103/00

(54) **STERILISATIONSSYSTEM FÜR BEHÄLTERBEHANDLUNGSANLAGE UND ZUGEHÖRIGES VERFAHREN**

(30) Priorität: 14.03.2025 DE 102025109986
(71) Anmelder: Krones AG, 93073 Neutraubling (DE)
(72) Erfinder: SCHAFACZEK, Bernd, 93073 Neutraubling (DE); SOELLNER, Juergen, 93073 Neutraubling (DE); STUBENHOFER, Markus, 93073 Neutraubling (DE); MEYER, Andreas, 93073 Neutraubling (DE)
(74) Vertreter: v. Bezold & Partner Patentanwälte - PartG mbB

(57) **Zusammenfassung**

Die Erfindung betrifft u.a. ein Sterilisationssystem (10) für eine Behälterbehandlungsanlage. Das Sterilisationssystem (10) weist ein Speicherbehältnis (12) für eine Wasserstoffperoxidlösung und eine Umkehrosmosevorrichtung (24) zum Behandeln der Wasserstoffperoxidlösung auf. Die Umkehrosmosevorrichtung (24) ist mit dem Speicherbehältnis (12) zum Empfangen der Wasserstoffperoxidlösung von dem Speicherbehältnis (12) verbunden. Das Sterilisationssystem (10) weist ferner einen Verdampfer (38), der mit der Umkehrosmosevorrichtung (24) zum Empfangen der Wasserstoffperoxidlösung von der Umkehrosmosevorrichtung (24) verbunden ist, und eine Sterilisierungsvorrichtung (40), die mit dem Verdampfer (38) zum Empfangen der verdampften Wasserstoffperoxidlösung verbunden ist, auf.

## Beschreibung

### Technisches Gebiet

Die Erfindung betrifft ein Sterilisationssystem für eine Behälterbehandlungsanlage, eine Behälterbehandlungsanlage sowie ein Verfahren zum Sterilisieren in einer Behälterbehandlungsanlage.

### Technischer Hintergrund

In einer Behälterbehandlungsanlage, wie bspw. einer Abfüllanlage, kann Wasserstoffperoxid als Desinfektionsmittel oder Reinigungsverstärker zum Einsatz kommen. Herkömmlich erfolgt die Bereitstellung von Wasserstoffperoxid in unterschiedlichen Qualitäten von diversen Lieferanten. Die unterschiedlichen Qualitäten der Wasserstoffperoxidlösung können sich insbesondere dadurch unterscheiden, dass ein unterschiedlich großer Anteil an Stabilisatoren in der Wasserstoffperoxidlösung vorhanden ist.

Die Menge an Stabilisatoren in der bereitgestellten Wasserstoffperoxidlösung kann einen großen Einfluss bei der Anwendung in einem Verdampfer eines Sterilisationssystems der Behälterbehandlungsanlage haben. Stabilisatoren können nämlich als Verdampfungsrückstände der verwendeten Wasserstoffperoxidlösung zurückbleiben. Die Rückstände legen sich an Düsen des Verdampfers, der Verdampferplatte oder der nachgelagerten Rohrleitung und Sensorik ab. Diese Rückstände können einen negativen Einfluss auf die Standzeit der eingesetzten Komponenten haben und zu einem erhöhten Wartungsaufwand und Stillstandzeiten der Behälterbehandlungsanlage führen.

Prinzipiell ist es daher wünschenswert, dass die herstellerseitig bereitgestellte Wasserstoffperoxidlösung nur einen geringen Anteil an Stabilisatoren aufweist. Die Handhabung, Lagerung und Transport einer solchen Wasserstoffperoxidlösung kann aufgrund des Mangels an Stabilisatoren jedoch nicht durch alle Hersteller und Lieferanten weltweit gewährleistet werden. Das führt dazu, dass eine optimal aufbereitete Wasserstoffperoxidlösung herstellerseitig nicht weltweit bereitgestellt werden kann. Hingegen sind Wasserstoffperoxidlösungen, die mit einem relativ großen Anteil an Stabilisatoren versetzt sind, weltweit verfügbar, über einen längeren Zeitraum lagerfähig und vergleichsweise günstig herzustellen.

Der Erfindung liegt die Aufgabe zu Grunde, eine verbesserte Technik zum Sterilisieren in einer Behälterbehandlungsanlage zu schaffen.

### Zusammenfassung der Erfindung

Die Aufgabe wird gelöst durch die Merkmale der unabhängigen Ansprüche. Vorteilhafte Weiterbildungen sind in den abhängigen Ansprüchen und der Beschreibung angegeben.

Ein Aspekt betrifft ein Sterilisationssystem für eine Behälterbehandlungsanlage. Das Sterilisationssystem weist ein Speicherbehältnis für eine Wasserstoffperoxidlösung auf. Das Sterilisationssystem weist ferner eine Umkehrosmosevorrichtung zum Behandeln der Wasserstoffperoxidlösung auf. Die Umkehrosmosevorrichtung ist mit dem Speicherbehältnis zum Empfangen der Wasserstoffperoxidlösung von dem Speicherbehältnis verbunden. Das Sterilisationssystem weist ferner einen Verdampfer zum Verdampfen der Wasserstoffperoxidlösung auf. Der Verdampfer ist mit der Umkehrosmosevorrichtung zum Empfangen der (behandelten) Wasserstoffperoxidlösung von der Umkehrosmosevorrichtung verbunden. Das Sterilisationssystem weist ferner eine Sterilisierungsvorrichtung zum Sterilisieren mittels der Wasserstoffperoxidlösung auf. Die Sterilisierungsvorrichtung ist mit dem Verdampfer zum Empfangen der verdampften Wasserstoffperoxidlösung verbunden.

Vorteilhaft ermöglicht das Sterilisationssystem, dass Stabilisatoren in der bereitgestellten Wasserstoffperoxidlösung direkt vor Ort in einer Behälterbehandlungsanlage mittels Umkehrosmose in der Umkehrosmosevorrichtung reduziert bzw. zurückgehalten werden können. Dadurch können die Stabilisatoren vorteilhaft erst kurz vor dem Verdampfen entfernt werden. Vorteilhaft lassen sich dadurch Verdampfungsrückstände aufgrund der Stabilisatoren auf ein Minimum reduzieren. Diese Reduzierung sorgt vorteilhaft für deutlich erhöhte Standzeiten der eingesetzten Komponenten bei der Anwendung in einem Verdampfer und nachgelagerten Komponenten, wie bspw. Sensoren und Rohrleitungen. Mit dem hierin vorgeschlagenen Sterilisationssystem können somit gleichbleibende Standzeiten unabhängig vom Hersteller der Wasserstoffperoxidlösung in diversen Ländern sichergestellt werden. Vor dem Entfernen der Stabilisatoren kann die stabilisierte Wasserstoffperoxidlösung vorteilhaft in dem Sammelbehältnis sicher gelagert werden. Erst wenn Wasserstoffperoxid für den Verdampfungsprozess tatsächlich benötigt wird, kann die Wasserstoffperoxidlösung über die Umkehrosmosevorrichtung gefahren und die Stabilisatoren aus der Lösung entfernt werden, bevor diese verdampft wird. Ein großer Vorteil kann somit darin bestehen, dass die Wasserstoffperoxidlösung während der Lagerung und des Transports mit Stabilisatoren versetzt sein kann, und es dennoch keine negativen Auswirkungen auf den Verdampfungsprozess gibt. Ein weiterer Vorteil kann in der geringen Baugröße der integrierten Umkehrosmosevorrichtung aufgrund des vergleichsweise geringen Volumenstroms liegen. Es wird nur der für den Betrieb tatsächlich erforderliche Volumenstrom durch die Umkehrosmosevorrichtung gefahren.

In einem Ausführungsbeispiel ist mindestens eines erfüllt von:
- die Umkehrosmosevorrichtung ist dazu ausgebildet, Stabilisatoren in der Wasserstoffperoxidlösung (z. B. mittels mindestens einer Membran) zurückzuhalten und die (stabilisatorverringerte) Wasserstoffperoxidlösung an den Verdampfer auszugeben, vorzugsweise als Permeat von der Umkehrosmosevorrichtung; und
- der Verdampfer ist mit einem Auslass, vorzugsweise Permeatauslass, der Umkehrosmosevorrichtung zum Empfangen der Wasserstoffperoxidlösung, vorzugsweise als Permeat von der Umkehrosmosevorrichtung, verbunden.

In einem weiteren Ausführungsbeispiel ist mindestens eines erfüllt von:
- die Umkehrosmosevorrichtung weist mindestens ein Umkehrosmose-Membranmodul auf; und
- die Umkehrosmosevorrichtung weist mindestens eine perforierte, vorzugsweise laserperforierte, Membran zum Zurückhalten von Stabilisatoren aus der Wasserstoffperoxidlösung auf.

In einer Ausführungsform ist mindestens eines erfüllt von:
- die Sterilisierungsvorrichtung ist zum Sterilisieren von Packmitteln, vorzugsweise Behältern (z. B. Dosen oder Flaschen oder Vorformlingen), (z. B. Behälter-) Verschlüssen oder Verpackungsbahnen ((z. B. Getränke-) Kartonbahnen oder Folienbahnen), ausgebildet; und
- die Sterilisierungsvorrichtung weist einen Packmittel-Förderer, vorzugsweise Behälterförderer oder Verschlussförderer oder Bahnförderer, zum Fördern der Packmittel durch die Sterilisierungsvorrichtung auf.

Vorteilhaft können auf diese Weise Packmittel in der Behälterbehandlungsanlage sicher mit der verdampften Wasserstoffperoxidlösung sterilisiert und desinfiziert werden, wobei Verdampfungsrückstände aufgrund der Nutzung der Umkehrosmosevorrichtung auf ein Minimum reduziert werden können.

In einer weiteren Ausführungsform weist das Sterilisationssystem ferner eine weitere Sterilisierungsvorrichtung auf, die mit einem (z. B. weiteren) Auslass, vorzugsweise Retentatauslass, der Umkehrosmosevorrichtung zum Empfangen der Wasserstoffperoxidlösung mit mittels der Umkehrosmosevorrichtung zurückgehaltenen Stabilisatoren, vorzugsweise als Retentat von der Umkehrosmosevorrichtung, verbunden ist. Eine Verwendung des Retentats des Umkehrosmoseprozesses für weitere Anwendungen hat den Vorteil, dass das Abfallprodukt der Umkehrosmose weiteren Prozessen zugeführt werden kann, die bspw. keine Verdampfung der Wasserstoffperoxidlösung benötigen, und keine hochwertig aufbereitete Wasserstoffperoxidlösung für diese Prozesse verwendet werden muss. Dadurch kann vorteilhaft der Verbrauch an aufbereiteter Wasserstoffperoxidlösung wesentlich reduziert werden.

In einer Ausführungsvariante ist die weitere Sterilisierungsvorrichtung dazu ausgebildet, die empfangene Wasserstoffperoxidlösung (mit den mittels der Umkehrosmosevorrichtung zurückgehaltenen Stabilisatoren) in eine Flüssigkeitsdichtung, vorzugsweise ein Wasserschloss, (z. B. einer Maschine der Behälterbehandlungsanlage) und/oder in eine Reinigungsflüssigkeit (z. B. einer Reinigungsvorrichtung der Behälterbehandlungsanlage) zuzuführen (z. B. einzudosieren), vorzugsweise ohne die Wasserstoffperoxidlösung zuvor zu verdampfen. Vorteilhaft können auf diese Weise Sterilisierungsanwendungen von der weiteren Sterilisierungsvorrichtung genutzt werden, die kein Verdampfen der Wasserstoffperoxidlösung benötigen und somit nicht durch Verdampfungsrückstände aufgrund der vorhandenen Stabilisatoren beeinträchtigt werden.

In einer weiteren Ausführungsvariante weist das Sterilisationssystem ferner mindestens eines auf von:
- ein, vorzugsweise vakuumbeaufschlagtes, Hebergefäß zum Abführen von Gasblasen aus der Wasserstoffperoxidlösung, wobei die Umkehrosmosevorrichtung über das Hebergefäß mit dem Speicherbehältnis verbunden ist;
- eine Zuführpumpe, wobei die Umkehrosmosevorrichtung über die Zuführpumpe mit dem Speicherbehältnis verbunden ist; und
- einen Partikelfilter, wobei die Umkehrosmosevorrichtung über den Partikelfilter mit dem Speicherbehältnis verbunden ist; und
- mindestens eine Vorfiltrationsstufe, die einen Trennfilter zum Herausfiltern von in der Wasserstoffperoxidlösung gelösten Stoffen aufweist, wobei die Umkehrosmosevorrichtung über die mindestens eine Vorfiltrationsstufe mit dem Speicherbehältnis verbunden ist.

Vorteilhaft kann auf diese Weise eine zuverlässige Zuführung einer gasblasenfreien und/oder partikelfreien Wasserstoffperoxidlösung zu der Umkehrosmosevorrichtung ermöglicht werden, um einen optimalen Betrieb der Umkehrosmosevorrichtung zu ermöglichen.

In einem Ausführungsbeispiel weist das Sterilisationssystem ferner eine Durchflusserfassungseinrichtung auf, die zum Erfassen eines Durchflusses der Wasserstoffperoxidlösung hin zu der Umkehrosmosevorrichtung angeordnet ist. Alternativ oder zusätzlich kann das Sterilisationssystem bspw. eine Sensoreinrichtung, vorzugsweise aufweisend einen Drucksensor und/oder einen Leitfähigkeitssensor, aufweisen, wobei die Sensoreinrichtung zum Erfassen der Wasserstoffperoxidlösung stromabwärts von der Umkehrosmosevorrichtung angeordnet ist. Optional kann das Sterilisationssystem ferner eine Diagnoseeinrichtung aufweisen, die dazu konfiguriert ist, ein Zusetzen oder Verblocken einer Membran der Umkehrosmosevorrichtung abhängig von einer Signalausgabe der Durchflusserfassungseinrichtung und/oder der Sensoreinrichtung zu erkennen. Vorteilhaft kann auf diese Weise eine nahende oder bereits vorhandene Fehlfunktion der Umkehrosmosevorrichtung automatisch erkannt und ggf. Gegenmaßnahmen eingeleitet werden.

Vorzugsweise kann die Diagnoseeinrichtung ferner dazu konfiguriert sein, zum Erkennen eines Zustands der Umkehrosmosevorrichtung und/oder des Zusetzens oder Verblockens der Membran der Umkehrosmosevorrichtung mit einer externen Recheninfrastruktur, vorzugsweise einer Cloud- Recheninfrastruktur, zu kommunizieren.

In einem weiteren Ausführungsbeispiel weist das Sterilisationssystem ferner mindestens eines auf von:
- eine Abführpumpe, wobei der Verdampfer über die Abführpumpe mit der Umkehrosmosevorrichtung verbunden ist;
- ein Rückschlagventil, vorzugsweise Rückschlagklappe, zum Verhindern eines Wasserstoffperoxidrückflusses zu der Umkehrosmosevorrichtung, wobei der Verdampfer über das Rückschlagventil mit der Umkehrosmosevorrichtung verbunden ist; und
- ein Sammelbehältnis, vorzugsweise Drucksammelbehältnis, zum Speichern der Wasserstoffperoxidlösung (z. B. druckbeaufschlagt), wobei der Verdampfer über das Sammelbehältnis mit der Umkehrosmosevorrichtung verbunden ist.

Vorteilhaft kann auf diese Weise eine zuverlässige Zuführung der behandelten Wasserstoffperoxidlösung zu dem Verdampfer ermöglicht werden.

In einer Ausführungsform weist das Sammelbehältnis einen Füllstandsensor zum Erfassen eines Füllstands in dem Sammelbehältnis auf. Optional kann das Sterilisationssystem ferner eine Steuereinrichtung aufweisen, die dazu konfiguriert ist, die Abführpumpe (und/oder die Zuführpumpe) abhängig von einer Signalausgabe des Füllstandsensors zu betreiben. Vorteilhaft kann auf diese Weise eine bedarfsgerechte Aufbereitung der Wasserstoffperoxidlösung mittels der Umkehrosmosevorrichtung nur in der tatsächlich erforderlichen Menge ermöglicht werden. Vorteilhaft kann dadurch das Sterilisationssystem und insbesondere dessen Umkehrosmosevorrichtung besonders kompakt ausgeführt werden.

Vorzugsweise kann sich der Begriff "Steuereinrichtung" auf eine Elektronik (z. B. ausgeführt als eine Treiberschaltung oder mit Mikroprozessor(en) und Datenspeicher) und/oder eine mechanische, pneumatische und/oder hydraulische Steuerung beziehen, die je nach Ausbildung Steuerungsaufgaben und/oder Regelungsaufgaben und/oder Verarbeitungsaufgaben übernehmen kann. Auch wenn hierin der Begriff "Steuern" verwendet wird, kann damit gleichsam zweckmäßig auch "Regeln" bzw. "Steuern mit Rückkopplung" und/oder "Verarbeiten" umfasst bzw. gemeint sein. Die Steuereinrichtung kann beispielsweise eine Zentralsteuereinrichtung sein oder mehrere dezentral bzw. verteilt angeordnete Steuereinheiten aufweisen.

In einer weiteren Ausführungsform ist mindestens eines erfüllt von:
- das Speicherbehältnis ist ein Fass oder ein Lagertank; und
- der Verdampfer weist mindestens eine Einsprühdüse für die Wasserstoffperoxidlösung (z. B. zum Einsprühen oder Vernebeln) und ein Verdampferelement, vorzugsweise Verdampferplatte, zum Verdampfen der Wasserstoffperoxidlösung auf.

Ein weiterer Aspekt betrifft eine Behälterbehandlungsanlage. Die Behälterbehandlungsanlage weist ein Sterilisationssystem wie hierin offenbart auf. Optional weist die Behälterbehandlungsanlage ferner einen, vorzugsweise aseptischen, Isolator (z. B. Reinraum) auf. Bevorzugt kann die Sterilisierungsvorrichtung an einer Einlassschleuse des Isolators angeordnet sein. Vorteilhaft können mit der Behälterbehandlungsanlage die gleichen Vorteile erzielt werden, die bereits unter Bezugnahme auf das Sterilisationssystem erläutert wurden.

Beispielsweise kann die Behälterbehandlungsanlage zum Temperieren, Herstellen, Reinigen, Beschichten, Prüfen, Abfüllen, Verschließen, Pasteurisieren, Dekorieren, Etikettieren, Bedrucken, Beschriften, Laserbeschriften und/oder Verpacken von Behältern für flüssige oder pastöse Medien, vorzugsweise Getränke, flüssige Nahrungsmittel oder Produkte aus der Pharma- oder Health-Care-Industrie, ausgebildet sein. Die Behälterbehandlungsanlage kann beispielsweise eine Getränkeabfüllanlage sein.

Beispielsweise können die Behälter als Flaschen, Dosen, Kanister, Kartons, Flakons, Phiolen, Tuben usw. ausgeführt sein.

Ein weiterer Aspekt betrifft ein Verfahren zum Sterilisieren, vorzugsweise mittels eines Sterilisationssystems wie hierin offenbart, in einer Behälterbehandlungsanlage, vorzugsweise wie hierin offenbart. Das Verfahren weist auf:
- Zuführen einer Wasserstoffperoxidlösung (z. B. mittels einer Zuführpumpe) von einem Speicherbehältnis zu einer Umkehrosmosevorrichtung, die mit dem Speicherbehältnis verbunden ist;
- Zurückhalten von Stabilisatoren in der Wasserstoffperoxidlösung mittels Umkehrosmose der Umkehrosmosevorrichtung,
- Verdampfen der Wasserstoffperoxidlösung von der Umkehrosmosevorrichtung, vorzugsweise als Permeat der Umkehrosmose, mittels eines Verdampfers (z. B. durch Versprühen der Wasserstoffperoxidlösung zu einem Verdampferelement des Verdampfers), der mit der Umkehrosmosevorrichtung verbunden ist; und
- Sterilisieren mittels der verdampften Wasserstoffperoxidlösung in einer Sterilisierungsvorrichtung, die mit dem Verdampfer verbunden ist, wobei vorzugsweise bei dem Sterilisieren Packmittel, bevorzugt Behälter (z. B. Dosen oder Flaschen oder Vorformlinge), (z. B. Behälter-) Verschlüsse oder Verpackungsbahnen ((z. B. Getränke-) Kartonbahnen oder Folienbahnen), sterilisiert werden.

Vorteilhaft können mit dem Verfahren die gleichen Vorteile erzielt werden, die hierin bereits unter Bezugnahme auf das Sterilisationssystem erläutert wurden. Selbiges gilt für die untenstehend erläuterten bevorzugten Ausführungsbeispiel des Verfahrens.

In einem Ausführungsbeispiel weist das Verfahren ferner auf: Zuführen der Wasserstoffperoxidlösung mit mittels der Umkehrosmosevorrichtung zurückgehaltenen Stabilisatoren, vorzugsweise als Retentat, von der Umkehrosmosevorrichtung zu einer weiteren Sterilisierungsvorrichtung (z. B. ohne Verdampfen der Wasserstoffperoxidlösung mit den zurückgehaltenen Stabilisatoren). Vorzugsweise kann die zugeführte Wasserstoffperoxidlösung in eine Flüssigkeitsdichtung, vorzugsweise ein Wasserschloss, (z. B. einer Maschine der Behälterbehandlungsanlage) und/oder in eine Reinigungsflüssigkeit (z. B. einer Reinigungsvorrichtung der Behälterbehandlungsanlage) zugeführt (z. B. eindosiert) werden, vorzugsweise ohne die Wasserstoffperoxidlösung zuvor zu verdampfen.

In einem weiteren Ausführungsbeispiel weist das Verfahren ferner mindestens eines auf von:
- Fördern des Packmittels mittels eines Packmittel-Förderers durch die Sterilisierungsvorrichtung während des Sterilisierens;
- Entfernen von Gasblasen aus der Wasserstoffperoxidlösung mittels eines, vorzugsweise vakuumbeaufschlagten, Hebergefäßes beim Zuführen der Wasserstoffperoxidlösung von dem Speicherbehältnis zu der Umkehrosmosevorrichtung;
- Filtern von Partikeln aus der Wasserstoffperoxidlösung mittels eines Partikelfilters beim Zuführen der Wasserstoffperoxidlösung von dem Speicherbehältnis zu der Umkehrosmosevorrichtung;
- Filtern von in der Wasserstoffperoxidlösung gelösten Stoffen mittels eines Trennfilters mindestens einer Vorfiltrationsstufe beim Zuführen der Wasserstoffperoxidlösung von dem Speicherbehältnis zu der Umkehrosmosevorrichtung;
- Erfassen eines Durchflusses der Wasserstoffperoxidlösung mittels einer Durchflusserfassungseinrichtung beim Zuführen der Wasserstoffperoxidlösung von dem Speicherbehältnis zu der Umkehrosmosevorrichtung und optional: Erkennen eines Zusetzens oder Verblockens der Umkehrosmosevorrichtung abhängig von dem erfassten Durchfluss mittels einer Diagnoseeinrichtung (z. B. in Kommunikation mit einer externen Recheninfrastruktur, vorzugsweise Cloud-Recheninfrastruktur);
- Erfassen eines Drucks und/oder eines elektrischen Leitwerts der Wasserstoffperoxidlösung mittels einer Sensoreinrichtung stromabwärts von der Umkehrosmosevorrichtung und optional: Erkennen eines Zusetzens oder Verblockens der Umkehrosmosevorrichtung abhängig von dem erfassten Druck und/oder elektrischen Leitwert mittels einer Diagnoseeinrichtung (z. B. in Kommunikation mit einer externen Recheninfrastruktur, vorzugsweise Cloud-Recheninfrastruktur);
- Verhindern eines Rückfließens der Wasserstoffperoxidlösung hin zu der Umkehrosmosevorrichtung mittels eines Rückschlagventils, wobei der Verdampfer über das Rückschlagventil mit der Umkehrosmosevorrichtung verbunden ist; und
- (z. B. druckbeaufschlagtes) Speichern der Wasserstoffperoxidlösung in einem Sammelbehältnis, vorzugsweise Drucksammelbehältnis, vor dem Verdampfen und optional: Erfassen eines Füllstands in dem Sammelbehältnis mittels eines Füllstandsensors und vorzugsweise davon abhängiges Betreiben einer Abführpumpe, die die Wasserstoffperoxidlösung von der Umkehrosmosevorrichtung zu dem Sammelbehältnis fördert.

Die zuvor beschriebenen bevorzugten Ausführungsformen und Merkmale der Erfindung sind beliebig miteinander kombinierbar.

### Kurzbeschreibung der Figuren

Weitere Einzelheiten und Vorteile der Erfindung werden im Folgenden unter Bezug auf die beigefügten Zeichnungen beschrieben. Es zeigen:
- Figur 1: eine schematische Darstellung eines beispielhaften Sterilisationssystems;
- Figur 2: eine schematische Darstellung eines weiteren beispielhaften Sterilisationssystems; und
- Figur 3: eine schematische Darstellung eines weiteren beispielhaften Sterilisationssystems.

Die in den Figuren gezeigten Ausführungsformen stimmen zumindest teilweise überein, so dass ähnliche oder identische Teile mit den gleichen Bezugszeichen versehen sind und zu deren Erläuterung auch auf die Beschreibung der anderen Ausführungsformen bzw. Figuren verwiesen wird, um Wiederholungen zu vermeiden.

### Detaillierte Beschreibung beispielhafter Ausführungsformen

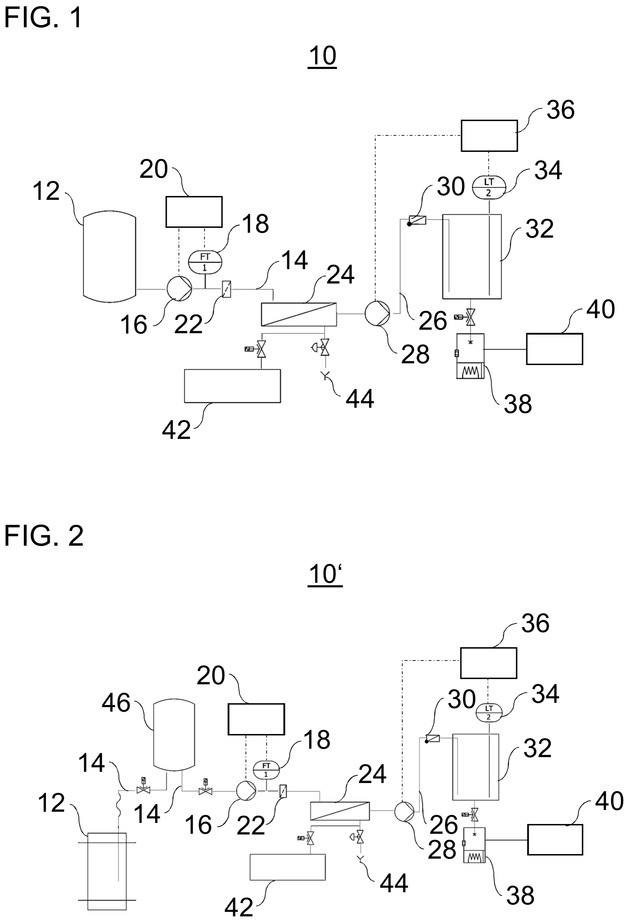

Die Figur 1 zeigt ein Sterilisationssystem 10 für eine Behälterbehandlungsanlage, z. B. Getränkeabfüllanlage.

Bevorzugt kann das Sterilisationssystem 10 in der Behälterbehandlungsanlage umfasst sein. Beispielsweise kann die Behälterbehandlungsanlage einen Isolator, z. B. Reinraum, aufweisen. Der Isolator ist bevorzugt aseptisch. Vorzugsweise kann der Isolator mit Sterilluft beaufschlagbar sein. Bevorzugt ist der Isolator derart mit Sterilluft beaufschlagbar, dass ein Luftdruck innerhalb des Isolators größer als in einer Umgebung des Isolators bzw. außerhalb des Isolators ist. Das Sterilisationssystem 10 kann mit dem Isolator verbunden sein, z. B. teilweise darin angeordnet sein.

Das Sterilisationssystem 10 weist ein Speicherbehältnis 12, eine Umkehrosmosevorrichtung 24, einen Verdampfer 38 und eine Sterilisierungsvorrichtung 40 auf. Vorzugsweise kann das Sterilisationssystem 10 ferner eine Zuführpumpe 16, eine Durchflusserfassungseinrichtung 18, eine Diagnoseeinrichtung 20, einen Partikelfilter 22, eine Abführpumpe 28, ein Rückschlagventil 30, ein Sammelbehältnis 32 und/oder eine weitere Sterilisationsvorrichtung 42 aufweisen.

Im Speicherbehältnis 12 kann eine Wasserstoffperoxidlösung gespeichert sein. Das Speicherbehältnis 12 kann beispielsweise ein Lagertank sein, wie in Figur 1 dargestellt ist. Alternativ kann das Speicherbehältnis 12 beispielsweise ein austauschbares Fass sein.

Die Wasserstoffperoxidlösung kann Wasserstoffperoxid und Stabilisatoren aufweisen, z. B. in einer wässrigen Lösung. Die Wasserstoffperoxidlösung kann in unterschiedlichen Reinheiten und/oder in unterschiedlichen Konzentrationen vorliegen.

Die Stabilisatoren können eine Stabilität der Wasserstoffperoxidlösung erhöhen. Die Stabilisatoren können anorganisch oder organisch sein. Die Stabilisatoren können einerseits die Eigenschaft aufweisen, wie eine organische Säure den pH-Wert zu puffern. Andererseits können die Stabilisatoren die Oberfläche katalytisch aktiver Metalle bedecken oder deren katalytisch aktive Ionen binden.

Als Stabilisatoren sind beispielsweise zu nennen: Zinnsäure oder Aluminiumoxid in kolloidalem Zustand, Natriumstannat und Natriumphosphat, Salze des Bariums, Alkalipyrophosphate und kolloidale Magnesiumverbindungen. Als wirksame stabilisierende organische Verbindungen können bspw. genannt werden: Salicylsäure, 8-Oxychinolin, Benzoesäure, Dipicolinsäure und Weinsäure. Häufig werden auch Mischungen aus anorganischen und organischen Stabilisatoren verwendet.

Das Speicherbehältnis 12 kann über eine Leitung 14 mit der Umkehrosmosevorrichtung 24 verbunden sein. Beispielsweise kann die Leitung 14 einen Auslass des Speicherbehältnis 12 mit einem Einlass der Umkehrosmosevorrichtung 24 verbinden. Es ist bspw. auch möglich, dass die Leitung 14 als ein Leitungssystem aus mehreren Leitungen aufgebaut ist.

Die Zuführpumpe 16 kann die Wasserstoffperoxidlösung von dem Speicherbehältnis 12 zu der Umkehrosmosevorrichtung 24 fördern bzw. zuführen. Über die Zuführpumpe 16 kann die Umkehrosmosevorrichtung 24 mit dem Speicherbehältnis 12 verbunden sein.

Die Zuführpumpe 16 kann jegliche zum Pumpen der Wasserstoffperoxidlösung geeignete Technik verwenden. Beispielsweise kann die Zuführpumpe 16 eine Drehkolbenpumpe oder eine Kreiselpumpe sein.

Die Zuführpumpe 16 kann stromaufwärts von der Umkehrosmosevorrichtung 24 angeordnet sein. Die Zuführpumpe 16 kann beispielsweise in der Leitung 14 angeordnet sein.

Die Durchflusserfassungseinrichtung 18 ist zum Erfassen eines Durchflusses der Wasserstoffperoxidlösung ausgebildet. Im Einzelnen kann die Durchflusserfassungseinrichtung 18 einen Durchfluss der Wasserstoffperoxidlösung hin zu der Umkehrosmosevorrichtung 24 erfassen.

Die Durchflusserfassungseinrichtung 18 kann bspw. mit der Leitung 14 verbunden sein. Bevorzugt kann die Durchflusserfassungseinrichtung 18 einen Durchfluss der Wasserstoffperoxidlösung durch die Leitung 14 erfassen. Beispielsweise kann die Durchflusserfassungseinrichtung 18 einen Durchfluss der Wasserstoffperoxidlösung stromabwärts von der Zuführpumpe 16 und/oder stromaufwärts von dem Partikelfilter 22 erfassen.

Beispielsweise kann die Durchflusserfassungseinrichtung 18 den Durchfluss messen, z. B. mengen-und/oder volumenmäßig. Die Durchflusserfassungseinrichtung 18 kann ein Messsignal, das den erfassten Durchfluss angibt, ausgeben, z. B. zu der Diagnoseeinrichtung 20.

Die Durchflusserfassungseinrichtung 18 kann jegliches geeignetes Durchflussmessprinzip zum Erfassen des Durchflusses aufweisen. Beispielsweise kann die Durchflusserfassungseinrichtung 18 eine magnetisch-induktive Durchflusserfassungseinrichtung oder eine Ultraschall-Durchflusserfassungseinrichtung sein.

Die Diagnoseeinrichtung 20 kann dazu konfiguriert sein, ein Zusetzen oder Verblocken einer Membran der Umkehrosmosevorrichtung 24 abhängig von einer Signalausgabe der Durchflusserfassungseinrichtung 18 zu erkennen.

Beispielsweise kann die Diagnoseeinrichtung 20 eine Signalausgabe von der Durchflusserfassungseinrichtung 18 empfangen. Die Signalausgabe kann einen von der Durchflusserfassungseinrichtung 18 erfassten Durchfluss der Wasserstoffperoxidlösung angegeben.

Beispielsweise kann die Diagnoseeinrichtung 20 mindestens einen Betriebsparameter bezüglich der Zuführpumpe 16 empfangen. Beispielsweise kann die Diagnoseeinrichtung den Betriebsparameter von der Zuführpumpe 16 oder einer Steuereinrichtung für die Zuführpumpe 16 empfangen. Der Betriebsparameter kann beispielsweise eine Förderleistung, eine Drehzahl und/oder ein Aktivierungszustand der Zuführpumpe 16 angeben.

Die Diagnoseeinrichtung 20 kann abhängig von dem Betriebsparameter der Zuführpumpe 16 und der Signalausgabe der Durchflusserfassungseinrichtung 18 ermitteln, ob der erfasste Durchfluss einem Soll-Durchfluss entspricht oder beispielsweise kleiner als der Soll-Durchfluss ist. Sofern der erfasste Durchfluss kleiner als der Soll-Durchfluss ist, kann dies ein Indiz dafür sein, dass eine Membran der Umkehrosmosevorrichtung 24 und/oder bspw. der Partikelfilter 22 zugesetzt oder verblockt ist.

Sofern die Diagnoseeinrichtung 20 ein Zusetzen oder Verblocken erkennt, kann die Diagnoseeinrichtung 20 beispielsweise einen Hinweis oder eine Warnung an eine Steuereinrichtung und/oder an eine Benutzerschnittstelle zum Ausgeben an einen Benutzer senden. Bspw. kann die Benutzerschnittstelle eine visuelle, akustische und/oder haptische/taktile Benutzerschnittstelle sein. Bevorzugt kann die Benutzerschnittstelle eine, vorzugsweise berührungsempfindliche, Anzeige, einen Lautsprecher, ein Mikrofon, eine Tastatur, einen Knopf und/oder mindestens eine Signalleuchte aufweisen. Beispielsweise kann nach dem Ausgeben des Hinweises / der Warnung eine manuelle oder automatische Reinigung oder ein Austausch einer Membran der Umkehrosmosevorrichtung 24 und/oder des Partikelfilters 22 durchgeführt werden.

Der Partikelfilter 22 kann zum Filtern von Partikeln und Verschmutzungen aus der Wasserstoffperoxidlösung ausgebildet sein. Der Partikelfilter 22 kann in der Leitung 14 angeordnet sein. Beispielsweise kann der Partikelfilter 22 stromabwärts von der Zuführpumpe 16 und/oder der Durchflusserfassungseinrichtung 18 angeordnet sein. Beispielsweise kann der Partikelfilter 22 stromaufwärts von der Umkehrosmosevorrichtung 24 angeordnet sein.

Die Umkehrosmosevorrichtung 24 ist mit dem Speicherbehältnis 12 zum Empfangen der Wasserstoffperoxidlösung von dem Speicherbehältnis 12 verbunden. Im Einzelnen kann die Umkehrosmosevorrichtung 24 über die Leitung 14 mit dem Speicherbehältnis 12 verbunden sein.

Die Umkehrosmosevorrichtung 24 kann bspw. mindestens ein Umkehrosmose-Membranmodul aufweisen.

Die Umkehrosmosevorrichtung 24 dient zum Behandeln der Wasserstoffperoxidlösung. Die Umkehrosmosevorrichtung 24 kann Stabilisatoren in der Wasserstoffperoxidlösung zurückhalten. Mittels der Zuführpumpe 16 kann ein Druck auf die Wasserstoffperoxidlösung ausgeübt werden. Druckbeaufschlagt kann die Wasserstoffperoxidlösung mindestens eine Membran der Umkehrosmosevorrichtung 24 passieren. Stabilsatoren können die mindestens eine Membran nicht passieren und werden somit von der mindestens einen Membran zurückgehalten. Der mittels der Zuführpumpe 16 ausgeübte Druck kann größer als ein osmotischer Druck für einen Konzentrationsausgleich sein, da bei der Umkehrosmose eine Richtung des osmotischen Flusses (zur Erlangung des osmotischen Gleichgewichts) umgekehrt werden kann.

Die mindestens eine Membran der Umkehrosmosevorrichtung 24 kann perforiert, vorzugsweise laserperforiert, sein. An der Perforation können die Stabilisatoren zurückgehalten werden bzw. die Stabilisatoren können die Perforation nicht passieren. Die Perforation kann so dimensioniert sein, dass die Stabilsatoren zurückgehalten werden können. Bevorzugt ist die mindestens eine Membran der Umkehrosmosevorrichtung 24 wasserstoffperoxidbeständig.

Die Umkehrosmosevorrichtung 24 kann die Wasserstoffperoxidlösung, die die mindestens eine Membran passiert hat, ausgeben. Diese Wasserstoffperoxidlösung kann auch als stabilisatorverringerte Wasserstoffperoxilösung oder als Permeat von der Umkehrosmosevorrichtung 24 bezeichnet werden.

Die Umkehrosmosevorrichtung 24 kann über eine Leitung 26 mit dem Sammelbehältnis 32 verbunden sein. Beispielsweise kann die Leitung 26 einen Auslass, vorzugsweise Permeatauslass, der Umkehrosmosevorrichtung 24 mit einem Einlass des Speicherbehältnis 32 verbinden. Es ist bspw. auch möglich, dass die Leitung 26 als ein Leitungssystem aus mehreren Leitungen aufgebaut ist.

Die Abführpumpe 28 kann die Wasserstoffperoxidlösung, die die mindestens eine Membran passiert hat, von der Umkehrosmosevorrichtung 24 abfördern.

Die Abführpumpe 28 kann jegliche zum Pumpen der Wasserstoffperoxidlösung geeignete Technik verwenden. Beispielsweise kann die Abführpumpe 28 eine Drehkolbenpumpe oder eine Kreiselpumpe sein.

Die Abführpumpe 28 kann stromabwärts von der Umkehrosmosevorrichtung 24 angeordnet sein. Bevorzugt kann die Abführpumpe 28 stromabwärts von einem Permeatauslass der Umkehrosmosevorrichtung 24 angeordnet sein. Bevorzugt kann die Abführpumpe 28 stromaufwärts von dem Rückschlagventil 30 angeordnet sein. Die Abführpumpe 28 kann beispielsweise in der Leitung 26 angeordnet sein.

Das Rückschlagventil 30 kann einen Rückfluss der Wasserstoffperoxidlösung zu der Umkehrosmosevorrichtung verhindern. Das Rückschlagventil 30 kann auch als ein Rückflussverhinderer bezeichnet werden. Bevorzugt ist das Rückschlagventil 30 als eine Rückschlagklappe ausgebildet.

Das Rückschlagventil 30 kann einen Fluss der Wasserstoffperoxidlösung nur in eine Richtung von der Umkehrosmosevorrichtung 24 zu dem Sammelbehältnis 32 erlauben. Das Rückschlagventil 30 kann sich bspw. öffnen, wenn ein Druck am Einlass des Rückschlagventils 30 höher ist als ein Druck am Auslass des Rückschlagventils 30. Dieser Druckunterschied kann einen Verschlussmechanismus (z.B. eine Kugel, eine Scheibe oder eine Klappe) öffnen, und die Wasserstoffperoxidlösung kann durchfließen. Wenn bspw. ein Druck am Einlass des Rückschlagventils 30 sinkt oder ein Druck am Auslass des Rückschlagventils 30 höher wird, kann sich des Rückschlagventil 30 selbsttätig schließen. Dies geschieht bspw. durch eine Feder, Schwerkraft oder ein Gegengewicht, das den Verschlussmechanismus zurück in die geschlossene Position drückt.

Bevorzugt ist das Rückschlagventil 30 stromabwärts von der Abführpumpe 28 und/oder stromaufwärts von dem Sammelbehältnis 32 angeordnet. Beispielsweise kann das Rückschlagventil 30 in der Leitung 26 angeordnet sein.

Das Sammelbehältnis 32 kann zum Speichern der Wasserstoffperoxidlösung von der Umkehrosmosevorrichtung 24 dienen.

Bevorzugt kann das Sammelbehältnis 32 stromabwärts von dem Rückschlagventil 30 und/oder der Abführpumpe 28 angeordnet sein. Beispielsweise kann die Leitung 26 in das Sammelbehältnis 32 münden.

Bevorzugt kann das Sammelbehältnis 32 stromaufwärts von dem Verdampfer 38 angeordnet sein. Beispielsweise kann ein Auslass des Sammelbehältnisses 32 und ein Einlass des Verdampfers 38 über eine Leitung miteinander verbunden sein. In dieser Leitung kann bspw. ein Steuerventil und/oder ein Absperrventil angeordnet sein.

Es ist möglich, dass die Wasserstoffperoxidlösung in dem Sammelbehältnis 32 unter Druck gespeichert wird. Der Druckaufbau kann mittels der Abführpumpe 28 erfolgen. Beispielsweise kann die Wasserstoffperoxidlösung mit einem Druck > 1 bar, z. B zwischen 1 bar und 2 bar, in dem Sammelbehältnis 32 unter Druck (druckbeaufschlagt) gespeichert werden. In einer solchen Ausführung kann das Sammelbehältnis 32 auch als ein Drucksammelbehältnis bezeichnet werden.

Das Sammelbehältnis 32 kann einen Füllstandsensor 34 aufweisen. Der Füllstandsensor 34 kann einen Füllstand der Wasserstoffperoxidlösung in dem Sammelbehältnis 32 erfassen. Der Füllstandsensor 34 kann ein Signal ausgegeben, das den erfassten Füllstand angibt. Das Signal kann beispielsweise von einer Steuereinrichtung 36 empfangen werden.

Die Steuereinrichtung 36 kann abhängig von der Signalausgabe des Füllstandsensors 34 einen Betrieb der Abführpumpe 28 und optional der Zuführpumpe 16 anpassen. Vorzugsweise kann die Steuereinrichtung 36 den Betrieb derart anpassen, dass in dem Sammelbehältnis 32 ein vorgegebener Mindestfüllstand nicht unterschritten und/oder ein vorgegebener Maximalfüllstand nicht überschritten wird.

Der Verdampfer 38 ist mit der Umkehrosmosevorrichtung 24 zum Empfangen der Wasserstoffperoxidlösung von der Umkehrosmosevorrichtung 24 verbunden. Im Einzelnen kann der Verdampfer 38 über das Sammelbehältnis 32, das Rückschlagventil 30 und/oder die Abführpumpe 28 mit einem Auslass, vorzugsweise Permeatauslass, der Umkehrosmosevorrichtung 24 verbunden sein.

Der Verdampfer 38 ist zum Verdampfen der empfangenen Wasserstoffperoxidlösung ausgebildet. Beispielsweise kann der Verdampfer 38 mindestens eine Einsprühdüse für die Wasserstoffperoxidlösung und ein Verdampferelement zum Verdampfen des eingesprühten Wasserstoffperoxids aufweisen. Das Verdampferelement kann beheizbar sein. Das Verdampferelement kann beispielsweise eine Verdampferplatte sein. Bevorzugt kann das Verdampferelement mittels eines elektrischen Heizelements beheizt werden.

Beispielsweise kann der Verdampfer 38 als ein sogenannter Flash-Verdampfer oder ein Nebel-Verdampfer ausgeführt sein.

Es ist möglich, dass der Verdampfer 38 mehrere, z. B. zwei, Verdampfungseinheiten aufweist. Die mehreren Verdampfungseinheiten können beispielsweise parallelgeschaltet sein.

Die Sterilisationsvorrichtung 40 ist mit dem Verdampfer 38 zum Empfangen der verdampften Wasserstoffperoxidlösung verbunden. Beispielsweise kann ein Einlass der Sterilisationsvorrichtung 40 mit einem Auslass des Verdampfers 38 verbunden sein.

Die Sterilisationsvorrichtung 40 dient zum Sterilisieren mittels der verdampften Wasserstoffperoxidlösung. Die verdampfte Wasserstoffperoxidlösung kann beispielsweise mit Luft gemischt zum Sterilisieren verwendet werden.

Bevorzugt ist die Sterilisierungsvorrichtung zum Sterilisieren von Packmitteln ausgebildet. Beispielsweise kann die Sterilisierungsvorrichtung 40 Behälter, wie beispielsweise Flaschen oder Dosen oder Vorformlinge, sterilisieren. Alternativ oder zusätzlich kann die Sterilisierungsvorrichtung 40 Verschlüsse, wie beispielsweise Behälterverschlüsse, sterilisieren. Alternativ oder zusätzlich kann die Sterilisierungsvorrichtung 40 beispielsweise Verpackungsbahnen, zum Beispiel Kartonbahnen oder Folienbahnen, sterilisieren.

Es ist möglich, dass die Sterilisierungsvorrichtung 40 mehrere Sterilisierungsmodule aufweist. Die Sterilisierungsmodule können beispielsweise verteilt in der Behälterbehandlungsanlage angeordnet. Beispielsweise kann ein Sterilisierungsmodul zum Sterilisieren von Behältern, ein Sterilisierungsmodul zum Sterilisieren von Verschlüssen und/oder ein Sterilisierungsmodul zum Sterilisieren von Verpackungsbahnen umfasst sein.

Bevorzugt weist die Sterilisierungsvorrichtung 40 einen Packmittel-Förderer auf. Der Packmittel-Förderer kann beispielsweise ein Behälterförderer oder ein Verschlussförderer oder ein Bahnförderer sein. Der Packmittel-Förderer kann ein Packmittel durch die Sterilisierungsvorrichtung 40 fördern. Die Sterilisierungsvorrichtung kann das Packmittel sterilisieren, während der Packmittel-Förderer das Packmittel durch die Sterilisierungsvorrichtung 40 fördert.

Beispielsweise kann die Sterilisierungsvorrichtung 40 an einer Einlassschleuse eines Isolators der Behälterbehandlungsanlage angeordnet sein. Beispielsweise kann der Packmittel-Förderer der Sterilisierungsvorrichtung 40 in der Einlassschleuse angeordnet sein oder die Einlassschleuse passieren.

Die weitere Sterilisationsvorrichtung 42 kann mit einem weiteren Auslass der Umkehrosmosevorrichtung 24 verbunden sein. Bevorzugt ist die weitere Sterilisationsvorrichtung 42 so mit der Umkehrosmosevorrichtung 24 verbunden, dass sie die Wasserstoffperoxidlösung mit den zurückgehaltenen Stabilisatoren von der Umkehrosmosevorrichtung 24 empfangen kann. Beispielsweise kann die weitere Sterilisationsvorrichtung 42 mit einem Retentatauslass der Umkehrosmosevorrichtung 24 verbunden sein. Beispielsweise kann die weitere Sterilisationsvorrichtung 42 ein Retentat der Umkehrosmose von der Umkehrosmosevorrichtung 24 empfangen.

Beispielsweise kann die weitere Sterilisationsvorrichtung 42 über eine Leitung mit dem weiteren Auslass der Umkehrosmosevorrichtung 24 verbunden sein. In dieser Leitung kann bspw. ein Steuerventil und/oder ein Absperrventil angeordnet sein.

Die weitere Sterilisierungsvorrichtung 42 kann dazu ausgebildet sein, die empfangene Wasserstoffperoxidlösung mit den zurückgehaltenen Sterilisatoren zum Sterilisieren zu verwenden, vorzugsweise ohne dass die Wasserstoffperoxidlösung hierzu verdampft wird. Beispielsweise kann die Wasserstoffperoxidlösung in eine Flüssigkeitsdichtung, vorzugsweise ein Wasserschloss, zugeführt werden, z. B. ausgemischt mit Wasser. Auch möglich ist, dass die weitere Sterilisationsvorrichtung 42 die Wasserstoffperoxidlösung beispielsweise als ein Booster in eine Reinigungsflüssigkeit zuführt. Die Reinigungsflüssigkeit kann zum Reinigen jeglicher Komponente der Behälterbehandlungsanlage genutzt werden, zum Beispiel bei einer manuellen oder automatischen Reinigung.

Es ist möglich, dass das Sterilisationssystem 10 einen Abfluss 44 aufweist. Der Abfluss 44 kann beispielsweise mit dem weiteren Auslass, vorzugsweise Retentatauslass, der Umkehrosmosevorrichtung 24 verbunden sein. Über den Abfluss 44 kann Wasserstoffperoxidlösung mit den zurückgehaltenen Stabilisatoren von der Umkehrosmosevorrichtung 24 abgelassen werden, zum Beispiel zur Probenentnahme oder zur anderweitigen Verwendung. Beispielsweise kann der Abfluss 44 über eine Leitung mit dem weiteren Auslass der Umkehrosmosevorrichtung 24 verbunden sein. In dieser Leitung kann bspw. ein Steuerventil und/oder ein Absperrventil angeordnet sein.

Die Figur 2 zeigt ein modifiziertes Sterilisationssystem 10'.

Im in Figur 2 dargestellten Ausführungsbeispiel ist das Speicherbehältnis 12 als ein Fass ausgeführt. Das Fass kann mit einer Wasserstoffperoxidlösung gefüllt sein. Das Fass kann bewegbar und austauschbar sein.

Das Sterilisationssystem 10' weist ein Hebergefäß 46 auf. Das Hebergefäß 46 kann zum Abführen / Entfernen von Gasblasen aus der Wasserstoffperoxidlösung ausgebildet sein. Beispielsweise kann das Hebergefäß 46 vakuumbeaufschlagt sein. Beispielsweise kann eine Vakuumquelle mit dem Hebergefäß 46 zum Absaugen von Gas aus dem Hebergefäß 46 verbunden sein.

Bevorzugt kann das Hebergefäß 46 stromabwärts von dem Speicherbehältnis 12 und/oder stromaufwärts von der Zuführpumpe 16, der Durchflusserfassungseinrichtung 18, dem Partikelfilter 22 und/oder der Umkehrosmosevorrichtung 24 angeordnet sein.

Ein Einlass des Hebergefäßes 46 kann beispielsweise über einen Abschnitt der Leitung 14 mit dem Speicherbehältnis 12 verbunden sein. In diesem Abschnitt der Leitung kann bspw. ein Steuerventil und/oder ein Absperrventil angeordnet sein.

Ein Auslass des Hebergefäßes 46 kann beispielsweise direkt mit einem Abschnitt der Leitung 14, in dem die Zuführpumpe 16, die Durchflusserfassungseinrichtung 18 und/oder der Partikelfilter 22 angeordnet ist, verbunden sein. Der Abschnitt der Leitung 14 kann den Auslass des Hebergefäßes 46 mit einem Einlass der Umkehrosmosevorrichtung 24 verbinden. In diesem Abschnitt der Leitung kann bspw. ein Steuerventil und/oder ein Absperrventil angeordnet sein, z. B. direkt stromabwärts von dem Auslass des Hebergefäßes 46 und/oder stromaufwärts von der Zuführpumpe 16.

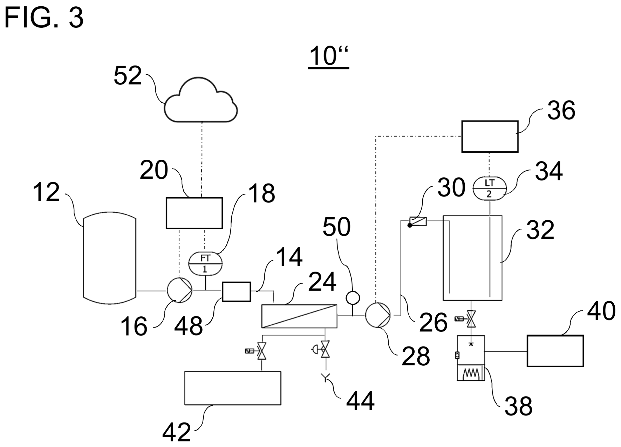

Die Figur 3 zeigt ein modifiziertes Sterilisationssystem 10", ähnlich zu dem Sterilisationssystem 10 von Figur 1.

Beispielsweise kann das Sterilisationssystem 10" bspw. mindestens eine Vorfiltrationsstufe 48 aufweisen. Die mindestens eine Vorfiltrationsstufe 48 kann bspw. mindestens einen Partikelfilter und/oder mindestens einen Trennfilter (z. B. Membranfilter) zum Herausfiltern von in der Wasserstoffperoxidlösung gelösten Stoffen, z. B. Fluiden, aufweisen.

Die mindestens eine Vorfiltrationsstufe 48 kann in der Leitung 14 angeordnet sein. Beispielsweise kann die mindestens eine Vorfiltrationsstufe 48 stromaufwärts oder stromabwärts von der Zuführpumpe 16 angeordnet sein. Beispielsweise kann die mindestens eine Vorfiltrationsstufe 48 stromaufwärts von der Umkehrosmosevorrichtung 24 angeordnet sein.

Vorzugsweise kann durch die mindestens eine Vorfiltrationsstufe 48 die Möglichkeit für eine Prozessfenstererweiterung geschaffen werden. Eine stärker verunreinigte Wasserstoffperoxidlösung kann durch die mindestens eine Vorfiltrationsstufe 48 für den Prozess einsetzbar gemacht werden. Es ist möglich, dass eine Funktionsfähigkeit der mindestens einen Vorfiltrationsstufe 48 überwacht wird, z. B. wie hierin für die Umkehrosmosevorrichtung 24 beschrieben ist.

Es ist auch möglich, dass das Sterilisationssystem 10" bspw. eine Sensoreinrichtung 50 aufweist. Die Sensoreinrichtung 50 kann zum Erfassen der Wasserstoffperoxidlösung stromabwärts von der Umkehrosmosevorrichtung 24 angeordnet sein. Beispielsweise kann die Sensoreinrichtung 50 in der Leitung 26 angeordnet sein.

Die Sensoreinrichtung 50 kann bspw. einen Drucksensor und/oder einen Leitfähigkeitssensor aufweisen. Der Drucksensor kann einen Druck der Wasserstoffperoxidlösung stromabwärts von der Umkehrosmosevorrichtung 24 erfassen. Der Leitfähigkeitssensor kann eine elektrische Leitfähigkeit der Wasserstoffperoxidlösung stromabwärts von der Umkehrosmosevorrichtung 24 erfassen. Die Sensoreinrichtung 50 kann ein Messsignal, das den erfassten Druck und/oder den erfassten elektrischen Leitwert angibt, ausgeben, z. B. zu der Diagnoseeinrichtung 20.

Auf Basis des erfassten elektrischen Leitwerts kann dann bspw. mittels der Diagnoseeinrichtung 20 geprüft werden, ob der erfasste elektrische Leitwert kleiner oder gleich einem vorgegebenen Leitfähigkeitsgrenzwert, z. B. ca. 10 µS/cm, ist. Zusätzlich oder alternativ kann mittels der Diagnoseeinrichtung 20 abhängig von dem erfassten Druck geprüft werden, ob für den erfassten Druck eine ausreichende Menge an Wasserstoffperoxid durch die Umkehrosmosevorrichtung 24 gedrückt wird oder nicht. Übersteigt bspw. der erfasste Druck einen vorgegebenen Druckgrenzwert, kann gefolgert werden, dass die Umkehrosmosevorrichtung 24 zugesetzt oder verblockt ist. Unterschreitet der erfasste Druck bspw. einen vorgegebenen (weiteren) Druckgrenzwert, kann gefolgert werden, dass eine Membran der Umkehrosmosevorrichtung 24 gebrochen ist.

Wie bereits erwähnt, kann die Diagnoseeinrichtung 20, wenn ein Zusetzen oder Verblocken erkannt wird, beispielsweise einen Hinweis oder eine Warnung an eine Steuereinrichtung und/oder an eine Benutzerschnittstelle zum Ausgeben an einen Benutzer senden. Die Benutzerschnittstelle kann bspw. ein Touchpanel, ein Smartphone oder ein anderes Smart Device sein. Beispielsweise kann nach dem Ausgeben des Hinweises / der Warnung eine manuelle oder automatische Reinigung (z. B. Rückspülung) oder ein Austausch einer Membran der Umkehrosmosevorrichtung 24 und/oder der mindestens einen Vorfiltrationsstufe 48 durchgeführt werden.

Ob eine Membran der Umkehrosmosevorrichtung 24 ausgetauscht werden muss, kann bspw. von den bisherigen Betriebsstunden und den erfassten Leitfähigkeitswerten nach einer Reinigung der Membran abhängen. Beispielsweise können ein vergleichsweise geringer Druck und eine vergleichsweise hohe elektrische Leitfähigkeit auf Schäden oder Brüche in der Membran hindeuten. Aus den Daten zum aktuellen Druck, Betriebsstunden und Leitfähigkeit kann einem Bediener über die Benutzerschnittstelle eine Empfehlung, was als nächstes zu tun ist (z. B. Rückspülung in 10 Stunden notwendig oder Austausch Membran in 100 Betriebsstunden), und/oder eine Angabe darüber, wie ein aktueller Zustand der Umkehrosmosevorrichtung 24 ist, ausgegeben werden. Es ist möglich, dass eine automatische Rückspülung bei einem geplanten Produktionstopp erfolgt.

Besonders bevorzugt kann die Diagnoseeinrichtung 20 zum Erkennen des Zusetzens oder Verblockens der Membran der Umkehrosmosevorrichtung 24 mit einer externen Recheninfrastruktur 52 kommunizieren. Die externe Recheninfrastruktur 52 kann bevorzugt eine Cloud- Recheninfrastruktur sein.

Beispielsweise können in der externen Recheninfrastruktur 52 KI-Algorithmen (z. B. supervised / unsupervised / reinforcement Machine Learning) angewendet werden, um einen Zustand der Umkehrosmosevorrichtung 24, z. B. ein Zusetzen oder Verblocken der Membran der Umkehrosmosevorrichtung 24, zu erkennen. Hierfür kann die externe Recheninfrastruktur 52 über die Diagnoseeinrichtung 20 bspw. die Signalausgabe der mindestens einen Vorfiltrationsstufe 48 und/oder der Sensoreinrichtung 50 empfangen. Bevorzugt kann die externe Recheninfrastruktur 52 auf große Datenmengen (Big Data) von einer Vielzahl von Sterilisationssystemen 10 zugreifen. Vorzugsweise kann die externe Recheninfrastruktur 52 eine, bevorzugt KI-gestützte, Mustererkennung und/oder eine Big Data-Analyse zur Anomalieerkennung und/oder Betriebsoptimierung durchführen. Beispielsweise kann die externe Rechnerinfrastruktur 52 ihre Ergebnisse an die Diagnoseeinrichtung 20 zurückmelden.

Es versteht sich, dass alle hierin unter Bezugnahme auf die Figuren 1 bis 3 erläuterten Merkmale miteinander kombinierbar sind.

Die Erfindung ist nicht auf die vorstehend beschriebenen bevorzugten Ausführungsbeispiele beschränkt. Vielmehr ist eine Vielzahl von Varianten und Abwandlungen möglich, die ebenfalls von dem Erfindungsgedanken Gebrauch machen und deshalb in den Schutzbereich fallen. Insbesondere beansprucht die Erfindung auch Schutz für den Gegenstand und die Merkmale der Unteransprüche unabhängig von den in Bezug genommenen Ansprüchen. Insbesondere sind die einzelnen Merkmale des unabhängigen Anspruchs 1 jeweils unabhängig voneinander offenbart. Zusätzlich sind auch die Merkmale der Unteransprüche unabhängig von sämtlichen Merkmalen des unabhängigen Anspruchs 1 und beispielsweise unabhängig von den Merkmalen bezüglich des Vorhandenseins und/oder der Konfiguration des Speicherbehältnisses, der Umkehrosmosevorrichtung, des Verdampfers und/oder der Sterilisierungsvorrichtung des unabhängigen Anspruchs 1 offenbart und beanspruchbar. Alle Bereichsangaben hierin sind derart offenbart und beanspruchbar zu verstehen, dass gleichsam alle in den jeweiligen Bereich fallenden Werte einzeln offenbart sind, z. B. auch als jeweils bevorzugte engere Außengrenzen des jeweiligen Bereichs.

### Bezugszeichenliste

- 10: Sterilisationssystem
- 12: Speicherbehältnis
- 14: Leitung
- 16: Zuführpumpe
- 18: Durchflusserfassungseinrichtung
- 20: Diagnoseeinrichtung
- 22: Partikelfilter
- 24: Umkehrosmosevorrichtung
- 26: Leitung
- 28: Abführpumpe
- 30: Rückschlagventil
- 32: Sammelbehältnis
- 34: Füllstandsensor
- 36: Steuereinrichtung
- 38: Verdampfer
- 40: Sterilisationsvorrichtung
- 42: weitere Sterilisationsvorrichtung
- 44: Abfluss
- 46: Hebergefäß
- 48: Vorfiltrationsstufe
- 50: Sensoreinrichtung
- 52: externe Recheninfrastruktur

## Patentansprüche

1. Sterilisationssystem (10) für eine Behälterbehandlungsanlage, wobei das Sterilisationssystem (10) aufweist:
ein Speicherbehältnis (12) für eine Wasserstoffperoxidlösung;
eine Umkehrosmosevorrichtung (24) zum Behandeln der Wasserstoffperoxidlösung, wobei die Umkehrosmosevorrichtung (24) mit dem Speicherbehältnis (12) zum Empfangen der Wasserstoffperoxidlösung von dem Speicherbehältnis (12) verbunden ist;
einen Verdampfer (38) zum Verdampfen der Wasserstoffperoxidlösung, wobei der Verdampfer mit der Umkehrosmosevorrichtung (24) zum Empfangen der Wasserstoffperoxidlösung von der Umkehrosmosevorrichtung (24) verbunden ist; und
eine Sterilisierungsvorrichtung (40) zum Sterilisieren mittels der Wasserstoffperoxidlösung, wobei die Sterilisierungsvorrichtung (40) mit dem Verdampfer (38) zum Empfangen der verdampften Wasserstoffperoxidlösung verbunden ist.

2. Sterilisationssystem (10) nach Anspruch 1, wobei mindestens eines erfüllt ist von:
die Umkehrosmosevorrichtung (24) ist dazu ausgebildet, Stabilisatoren in der Wasserstoffperoxidlösung zurückzuhalten und die Wasserstoffperoxidlösung an den Verdampfer (38) auszugeben, vorzugsweise als Permeat von der Umkehrosmosevorrichtung (24);
der Verdampfer (38) ist mit einem Auslass, vorzugsweise Permeatauslass, der Umkehrosmosevorrichtung (24) zum Empfangen der Wasserstoffperoxidlösung, vorzugsweise als Permeat von der Umkehrosmosevorrichtung (24), verbunden.

3. Sterilisationssystem (10) nach Anspruch 1 oder Anspruch 2, wobei mindestens eines erfüllt ist von:
die Umkehrosmosevorrichtung (24) weist mindestens ein Umkehrosmose-Membranmodul auf; und
die Umkehrosmosevorrichtung (24) weist mindestens eine perforierte, vorzugsweise laserperforierte, Membran zum Zurückhalten von Stabilisatoren aus der Wasserstoffperoxidlösung auf.

4. Sterilisationssystem (10) nach einem der vorherigen Ansprüche, wobei mindestens eines erfüllt ist von:
die Sterilisierungsvorrichtung (40) ist zum Sterilisieren von Packmitteln, vorzugsweise Behältern, Verschlüssen oder Verpackungsbahnen, ausgebildet; und
die Sterilisierungsvorrichtung (40) weist einen Packmittel-Förderer, vorzugsweise Behälterförderer oder Verschlussförderer oder Bahnförderer, zum Fördern der Packmittel durch die Sterilisierungsvorrichtung (40) auf.

5. Sterilisationssystem (10) nach einem der vorherigen Ansprüche, ferner aufweisend:
eine weitere Sterilisierungsvorrichtung (42), die mit einem Auslass, vorzugsweise Retentatauslass, der Umkehrosmosevorrichtung (24) zum Empfangen der Wasserstoffperoxidlösung mit mittels der Umkehrosmosevorrichtung (24) zurückgehaltenen Stabilisatoren, vorzugsweise als Retentat von der Umkehrosmosevorrichtung (24), verbunden ist.

6. Sterilisationssystem (10) nach Anspruch 5, wobei:
die weitere Sterilisierungsvorrichtung (42) dazu ausgebildet ist, die empfangene Wasserstoffperoxidlösung in eine Flüssigkeitsdichtung, vorzugsweise ein Wasserschloss, und/oder in eine Reinigungsflüssigkeit zuzuführen.

7. Sterilisationssystem (10) nach einem der vorherigen Ansprüche, ferner aufweisend mindestens eines von:
ein, vorzugsweise vakuumbeaufschlagtes, Hebergefäß (46) zum Abführen von Gasblasen aus der Wasserstoffperoxidlösung, wobei die Umkehrosmosevorrichtung (24) über das Hebergefäß (46) mit dem Speicherbehältnis (12) verbunden ist;
eine Zuführpumpe (16), wobei die Umkehrosmosevorrichtung (24) über die Zuführpumpe (16) mit dem Speicherbehältnis (12) verbunden ist;
einen Partikelfilter (22), wobei die Umkehrosmosevorrichtung (24) über den Partikelfilter (22) mit dem Speicherbehältnis (12) verbunden ist; und
mindestens eine Vorfiltrationsstufe (48), die einen Trennfilter zum Herausfiltern von in der Wasserstoffperoxidlösung gelösten Stoffen aufweist, wobei die Umkehrosmosevorrichtung (24) über die mindestens eine Vorfiltrationsstufe (48) mit dem Speicherbehältnis (12) verbunden ist.

8. Sterilisationssystem (10) nach einem der vorherigen Ansprüche, ferner aufweisend mindestens eines von:
eine Durchflusserfassungseinrichtung (18), die zum Erfassen eines Durchflusses der Wasserstoffperoxidlösung hin zu der Umkehrosmosevorrichtung (24) angeordnet ist; und
eine Sensoreinrichtung (50), vorzugsweise aufweisend einen Drucksensor und/oder einen Leitfähigkeitssensor, wobei die Sensoreinrichtung (50) zum Erfassen der Wasserstoffperoxidlösung stromabwärts von der Umkehrosmosevorrichtung (24) angeordnet ist.

9. Sterilisationssystem (10) nach Anspruch 8, ferner aufweisend:
eine Diagnoseeinrichtung (20), die dazu konfiguriert ist, ein Zusetzen oder Verblocken einer Membran der Umkehrosmosevorrichtung (24) abhängig von einer Signalausgabe der Durchflusserfassungseinrichtung (18) und/oder der Sensoreinrichtung (50) zu erkennen,
wobei vorzugsweise:
die Diagnoseeinrichtung (20) ferner dazu konfiguriert ist, zum Erkennen eines Zustands der Umkehrosmosevorrichtung (24) und/oder des Zusetzens oder Verblockens der Membran der Umkehrosmosevorrichtung (24) mit einer externen Recheninfrastruktur (52), vorzugsweise einer Cloud- Recheninfrastruktur, zu kommunizieren.

10. Sterilisationssystem (10) nach einem der vorherigen Ansprüche, ferner aufweisend mindestens eines von:
eine Abführpumpe (28), wobei der Verdampfer (38) über die Abführpumpe (28) mit der Umkehrosmosevorrichtung (24) verbunden ist;
ein Rückschlagventil (30), vorzugsweise Rückschlagklappe, zum Verhindern eines Wasserstoffperoxidrückflusses zu der Umkehrosmosevorrichtung (24), wobei der Verdampfer (38) über das Rückschlagventil mit der Umkehrosmosevorrichtung (24) verbunden ist; und
ein Sammelbehältnis (32), vorzugsweise Drucksammelbehältnis, zum Speichern der Wasserstoffperoxidlösung, wobei der Verdampfer (38) über das Sammelbehältnis (32) mit der Umkehrosmosevorrichtung (24) verbunden ist.

11. Sterilisationssystem (10) nach Anspruch 10, wobei:
das Sammelbehältnis (32) einen Füllstandsensor (34) zum Erfassen eines Füllstands in dem Sammelbehältnis (32) aufweist; und optional
das Sterilisationssystem (10) eine Steuereinrichtung (36) aufweist, die dazu konfiguriert ist, die Abführpumpe (28) abhängig von einer Signalausgabe des Füllstandsensors (34) zu betreiben.

12. Sterilisationssystem (10) nach einem der vorherigen Ansprüche, wobei mindestens eines erfüllt ist:
das Speicherbehältnis (12) ist ein Fass oder ein Lagertank; und
der Verdampfer (38) weist mindestens eine Einsprühdüse für die Wasserstoffperoxidlösung und ein Verdampferelement, vorzugsweise Verdampferplatte, zum Verdampfen der Wasserstoffperoxidlösung auf.

13. Behälterbehandlungsanlage aufweisend:
ein Sterilisationssystem (10) nach einem der vorherigen Ansprüche; und optional
einen, vorzugsweise aseptischen, Isolator, wobei bevorzugt die Sterilisierungsvorrichtung (40) an einer Einlassschleuse des Isolators angeordnet ist.

14. Verfahren zum Sterilisieren, vorzugsweise mittels eines Sterilisationssystems (10) nach einem der Ansprüche 1 bis 12, in einer Behälterbehandlungsanlage, vorzugsweise nach Anspruch 13, wobei das Verfahren aufweist:
Zuführen einer Wasserstoffperoxidlösung von einem Speicherbehältnis (12) zu einer Umkehrosmosevorrichtung (24), die mit dem Speicherbehältnis (12) verbunden ist;
Zurückhalten von Stabilisatoren in der Wasserstoffperoxidlösung mittels Umkehrosmose der Umkehrosmosevorrichtung (24),
Verdampfen der Wasserstoffperoxidlösung von der Umkehrosmosevorrichtung (24), vorzugsweise als Permeat der Umkehrosmose, mittels eines Verdampfers (38), der mit der Umkehrosmosevorrichtung (24) verbunden ist; und
Sterilisieren mittels der verdampften Wasserstoffperoxidlösung in einer Sterilisierungsvorrichtung (40), die mit dem Verdampfer (38) verbunden ist, wobei vorzugsweise bei dem Sterilisieren Packmittel, bevorzugt Behälter, Verschlüsse oder Verpackungsbahnen, sterilisiert werden.

15. Verfahren nach Anspruch 14, ferner aufweisend:
Zuführen der Wasserstoffperoxidlösung mit mittels der Umkehrosmosevorrichtung (24) zurückgehaltenen Stabilisatoren, vorzugsweise als Retentat, von der Umkehrosmosevorrichtung (24) zu einer weiteren Sterilisierungsvorrichtung (42), die vorzugsweise die zugeführte Wasserstoffperoxidlösung in eine Flüssigkeitsdichtung, vorzugsweise ein Wasserschloss, und/oder in eine Reinigungsflüssigkeit zuführt.

16. Verfahren nach Anspruch 14 oder Anspruch 15, ferner aufweisend mindestens eines von:
Fördern des Packmittels mittels eines Packmittel-Förderers durch die Sterilisierungsvorrichtung (40) während des Sterilisierens;
Entfernen von Gasblasen aus der Wasserstoffperoxidlösung mittels eines, vorzugsweise vakuumbeaufschlagten, Hebergefäßes (46) beim Zuführen der Wasserstoffperoxidlösung von dem Speicherbehältnis (12) zu der Umkehrosmosevorrichtung (24);
Filtern von Partikeln aus der Wasserstoffperoxidlösung mittels eines Partikelfilters (22) beim Zuführen der Wasserstoffperoxidlösung von dem Speicherbehältnis (12) zu der Umkehrosmosevorrichtung (24);
Filtern von in der Wasserstoffperoxidlösung gelösten Stoffen mittels eines Trennfilters mindestens einer Vorfiltrationsstufe (48) beim Zuführen der Wasserstoffperoxidlösung von dem Speicherbehältnis (12) zu der Umkehrosmosevorrichtung (24);
Erfassen eines Durchflusses der Wasserstoffperoxidlösung mittels einer Durchflusserfassungseinrichtung (18) beim Zuführen der Wasserstoffperoxidlösung von dem Speicherbehältnis (12) zu der Umkehrosmosevorrichtung (24) und optional: Erkennen eines Zusetzens oder Verblockens der Umkehrosmosevorrichtung (24) abhängig von dem erfassten Durchfluss mittels einer Diagnoseeinrichtung (20);
Erfassen eines Drucks und/oder eines elektrischen Leitwerts der Wasserstoffperoxidlösung mittels einer Sensoreinrichtung (50) stromabwärts von der Umkehrosmosevorrichtung (24) und optional: Erkennen eines Zusetzens oder Verblockens der Umkehrosmosevorrichtung (24) abhängig von dem erfassten Druck und/oder elektrischen Leitwert mittels einer Diagnoseeinrichtung (20);
Verhindern eines Rückfließens der Wasserstoffperoxidlösung hin zu der Umkehrosmosevorrichtung (24) mittels eines Rückschlagventils (30), wobei der Verdampfer (38) über das Rückschlagventil (30) mit der Umkehrosmosevorrichtung (24) verbunden ist; und
Speichern der Wasserstoffperoxidlösung in einem Sammelbehältnis (32), vorzugsweise Drucksammelbehältnis, vor dem Verdampfen und optional: Erfassen eines Füllstands in dem Sammelbehältnis (32) mittels eines Füllstandsensors (34) und vorzugsweise davon abhängiges Betreiben einer Abführpumpe (28), die die Wasserstoffperoxidlösung von der Umkehrosmosevorrichtung (24) zu dem Sammelbehältnis (32) fördert.
